(19) Europäisches Patentamt
European Patent Office
Office européen des brevets

(11) EP 0 584 664 B1

(12) **EUROPÄISCHE PATENTSCHRIFT**

(45) Veröffentlichungstag und Bekanntmachung des Hinweises auf die Patenterteilung:
**04.11.1998 Patentblatt 1998/45**

(51) Int Cl.[6]: **C07K 5/06**, C07K 1/06, C08F 20/60 // B01D15/08

(21) Anmeldenummer: **93112946.4**

(22) Anmeldetag: **12.08.1993**

(54) **Polymerisierbare Dipeptide:Synthese, Polymerisation und Verwendung der Polymere zur chromatographischen Enantiomerentrennung**

Polymerizable dipeptides: preparation, polymerization and use of the polymers for the chromatographic separation of enantiomers

Dipeptides polymerisables: préparation, polymérisation et utilisation de ces polymères pour la séparation chromatographique d'enantiomères

(84) Benannte Vertragsstaaten:
**AT BE CH DE DK ES FR GB GR IE IT LI LU MC NL PT SE**

(30) Priorität: **25.08.1992 DE 4228135**

(43) Veröffentlichungstag der Anmeldung:
**02.03.1994 Patentblatt 1994/09**

(73) Patentinhaber: **BAYER AG**
**51368 Leverkusen (DE)**

(72) Erfinder:
- **Lange, Walter, Dr.**
  **D-50723 Köln (DE)**
- **Grosse-Bley, Michael, Dr.**
  **D-51061 Köln (DE)**
- **Bömer, Bruno, Dr.**
  **D-51467 Bergisch Gladbach (DE)**
- **Grosser, Rolf, Dr.**
  **D-51373 Leverkusen (DE)**
- **Hoever, Franz-Peter, Dr.**
  **D-51069 Köln (DE)**

(56) Entgegenhaltungen:
**EP-A- 0 379 917** **EP-A- 0 464 488**

- **DIE MAKROMOLEKULARE CHEMIE, Band 178, Nr. 8, August 1977, H THIG & WEPF VERLAG, Basel, Schweiz J. KOPECEK "Reactive Copolymers of N-(2-Hydroxypropyl)- methacrylamide with N-Metha- cryloylated derivatives of L-Leucine and L-Phenyl- alanine,1", Seiten 2169-2183**
- **CHEMICAL ABSTRACTS, Band 92, Nr. 17, 28. April 1980, Columbus, Ohio, USA BROWN E. et al. "The design of new synthetic carriers for affinity chromatography", Seite 229, Spalte 1, Zusammenfassung-Nr. 142 561g**

EP 0 584 664 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue optisch aktive polymerisierbare Dipeptide, Verfahren zu ihrer Herstellung, ihre Polymerisation und die Verwendung der Polymere als Adsorbentien für die chromatographische Trennung von Enantiomeren.

Die Trennung von Wirkstoff-Enantiomeren stellt eine wichtige Aufgabe dar, weil die einzelnen Enantiomere häufig unterschiedliche biologische Wirkungen und Nebenwirkungen aufweisen. Die Auftrennung kann vorteilhaft durch Chromatographie an chiralen stationären Phasen bzw. Adsorbentien erfolgen.

Es sind bereits eine Reihe von Adsorbentien bekannt (G. Gübitz, Chromatographia 30 (1990), 555-564; EP 249 078; EP 379 917), die zur chromatographischen Trennung von Wirkstoff-Enantiomeren geeignet sind. Dennoch kommt es immer wieder vor, daß die Auftrennung eines Wirkstoff-Racemats an den bekannten Adsorbentien nicht oder nur schlecht gelingt.

Es wurde nun überraschend gefunden, daß die neuen erfindungsgemäßen Adsorbentien für eine Reihe von Wirkstoff-Racematen eine sehr hohe Selektivität aufweisen, die eine deutlich effizientere Auftrennung als an den bisher bekannten Adsorbentien ermöglicht. Damit sind entweder höhere Raum-Zeit-Ausbeuten bei präparativen Trennungen oder eine verbesserte Enantiomerenanalytik z.B. von biologischen Analyten möglich.

Die Erfindung betrifft daher polymerisierbare optisch aktive Dipeptide der Formel (I)

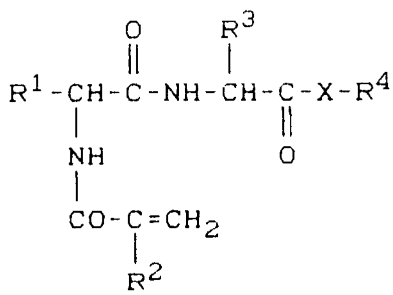

(I)

in der

- $R^1$ und $R^3$ gleich oder verschieden sind und für $C_1$-$C_5$-Alkyl, $CH_2$-O-A, $CH_2$-S-A, $(CH_2)_2$-S-$CH_3$, $CH_2$-Cyclohexyl, Cyclohexyl, Phenyl, Benzyl, 4-A-O-Benzyl, $(CH_2)_2$-Benzyl, Indolyl, $CH_2$-Naphthyl oder Naphthyl stehen, wobei A Wasserstoff, Methyl, t-Butyl oder Benzyl ist,
- $R^2$ Wasserstoff, Methyl oder Fluor bedeutet,
- X für Sauerstoff oder $NR^5$ steht,

wobei $R^5$ Wasserstoff, Methyl, Ethyl ist oder zusammen mit $R^4$ einen $C_5$-$C_6$-Cycloalkyl-Rest bildet und $R^4$ für einen geradkettigen oder verzweigten $C_3$-$C_{18}$-Alkyl- oder einen ein- bis vierfach $C_1$-$C_4$-alkylsubstituierten $C_3$-$C_{12}$-Cycloalkylrest, Benzyl, 1-Phenylethyl oder für ein- bis zweifach durch Fluor, Chlor, Trifluormethyl, Methoxy oder $C_1$-$C_4$-alkyl substituiertes Phenyl steht.

Bevorzugte Verbindungen der Formel (I) sind solche, in denen $R^1$, $R^3$ und $R^5$ die angegebene Bedeutung haben, $R^2$ Wasserstoff oder Methyl bedeutet und $R^4$ für $C_3$-$C_8$-Alkyl (wie z.B. 2-Propyl, 3-Pentyl, t-Butyl, Neopentyl), $C_5$-$C_z$-Cycloalkyl (Cyclopentyl, Cyclohexyl, Cycloheptyl), bis zu vierfach $C_1$-$C_4$-alkylsubstituiertes $C_6$-Cycloalkyl (wie Menthyl, Bornyl, Fenchyl), Benzyl, 1-Phenylethyl, Phenyl, 4-t-Butylphenyl, 3,5-Dichlorphenyl oder 3,5-Dimethylphenyl steht.

Wenn X nicht 0 sondern $NR^5$ bedeutet, dann kann $R^4$ auch für Methyl und Ethyl stehen oder mit $R^5$ einen $C_5$-$C_6$-Ring bilden.

Ist $R^4$ ein chiraler Rest, so wird er vorteilhaft in optisch aktiver Form verwendet.

Besonders bevorzugt sind Dipeptide der Formel (I), die sich von den Aminosäuren Alanin, Aminobuttersäure, Valin, Norvalin, Leucin, Isoleucin, Terleucin, Norleucin, Neopentylglycin, Serin, Cystein, Methionin, Hexahydrophenylalanin, Hexahydrophenylglycin, Phenylglycin, Phenylalanin, Tyrosin, Homophenylalanin, Tryptophan, Naphthylalanin oder Naphthylglycin ableiten.

Als besonders bevorzugte Verbindungen seien beispielhaft genannt:

N-Methacryloyl-S-Leu-S-Ile-O-t-butylester, N-Methacryloyl-S-Leu-S-Phe-d-menthylester, N-Methacryloyl-S-Leu-S-Leu-3-pentylamid, N-Methacryloyl-S-Val-S-Val-t-butylamid, N-Acryloyl-S-Phe-S-Phe-2-propylester, N-Methacryloyl-S-Val-S-Ala-l-bornylester, N-Methacryloyl-S-Ala-S-Met-3-pentylamid, N-Methacryloyl-R-Phg-S-Val-t-butylester, N-Methacryloyl-S-Leu-R-Phe-1-menthylester, N-Methacryloyl-S-Ala-R-(S)-methyl-Cys-diethylamid, N-Methacryloyl-S-Ala-S-Leu-d-menthylester, N-Methacryloyl-S-Phg-S-Val-t-butylester, N-Methacryloyl-S-Leu-S-Ile-3-pentylester, N-Methacryloyl-S-Phg-S-Met-diethylamid, N-Methacryloyl-S-Leu-R-hexahydrophenylglycin-diethylamid, N-Methacryloyl-S-Nle-S-Ile-t-butylester.

Die erfindungsgemäßen Verbindungen der Formel (I) werden hergestellt, indem man

A) Dipeptidderivate der Formel (II)

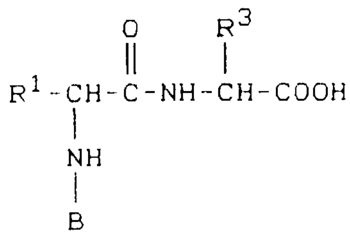

(II)

in welcher

$R^1$ und $R^3$ die oben angegebene Bedeutung haben und

B für eine in der Peptidchemie übliche, leicht abzuspaltende Gruppe steht,

mit in der Peptidchemie üblichen Verknüpfungsreaktionen mit dem Rest $XR^4$ verknüpft
wobei X und $R^4$ die oben angegebene Bedeutung haben
und anschließend den Rest B mit in der Peptidchemie üblichen Methoden abspaltet und die dann erhaltenen Verbindungen der allgemeinen Formel (III)

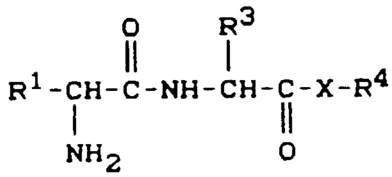

(III)

gegebenenfalls in Form ihrer Säureadditionsprodukte mit Acryloylderivaten der Formel (IV)

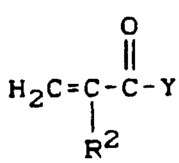

(IV)

in welcher

$R^2$ die oben angegebene Bedeutung hat und
Y für Fluor, Chlor, Brom oder für den Rest $-OCO-CR^2=CH_2$ steht,

in Gegenwart eines säurebindenden Mittel in inerten organischen Lösungsmitteln zu Verbindungen der Formel (I) umsetzt, oder
B) Aminosäurederivate der Formel (V)

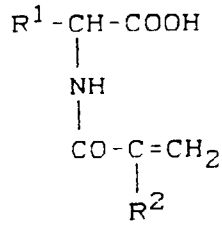

(V)

in welcher

$R^1$ und $R^2$ die oben angegebene Bedeutung haben, mit einem zweiten Aminosäurederivat der Formel (VI)

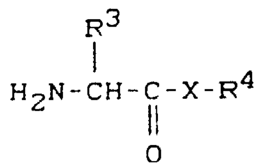

(VI)

in welcher

$R^3$, Y und $R^4$die oben angegebene Bedeutung haben,

nach üblichen Methoden zu Dipeptiden der Formel (I) verknüpft.

Bevorzugte Ausführungsform der Verfahrensvariante A ist die Verwendung von Verbindungen der Formel (II), in welcher B für leicht abzuspaltende Gruppen wie t-Butoxycarbonyl (BOC) oder Carbobenzoxycarbonyl (CBZ) steht.

Die Einführung des Restes $XR^4$ stellt eine in der Peptidchemie übliche Verknüpfungsreaktion dar. Zum Beispiel reagieren Amine entweder nach Aktivierung der Carbonsäurefunktion mit Basen wie Triethylamin oder N-Methylmorpholin und Chlorameisensäureester, mit Aktivestern über N-Hydroxysuccinimid (HOSU) und Dicyclohexylcarbodiimid (DCC) oder durch Zugabe von EEDQ (1-ELhoxycarbonyl-2-ethoxy-1,2-dihydrochinolin). Alkoholreste können z.B. säurekatalysiert (HCl) oder mit wasserabspaltenden Reagenzien wie z.B. Thionylchlorid oder DCC eingeführt werden; dazu arbeitet man in inerten organischen Lösungsmitteln wie z.B. Tetrahydrofuran, Dichlormethan, Toluol oder t-Butylmethylether.

Der Rest B wird im Falle BOC vorzugsweise mit HCl oder Trifluoressigsäure und im Falle CBZ vorzugsweise hydrogenolytisch abgespalten.

Die Umsetzung von III mit IV erfolgt vorzugsweise in Gegenwart eines säurebindenden Mittels wie z.B. Triethylamin oder Natronlauge. Als inerte Lösungsmittel werden beispielsweise eingesetzt Dichlormethan, Toluol oder t-Butylmethylether.

Bei der Umsetzung gemäß Verfahrensvariante B erfolgt die Kupplung von V mit VI vorzugsweise durch Aktivierung mit Basen wie Triethylamin oder N-Methylmorpholin und Chlorameisensäureester, durch Herstellung eines Aktivesters mit HOSU und DCC, oder durch Zugabe von EEDQ in einem inerten organischen Lösungsmittel wie z.B. Tetrahydrofuran, Dichlormethan oder t-Butylmethylether.

Die Erfindung betrifft auch die durch Polymerisation bzw. Copolymerisation der optisch aktiven Dipeptide der Formel (I) erhältlichen optisch aktiven Polymere und Copolymere, die mindestens 40 Mol-%, vorzugsweise mindestens 50 Mol-% Struktureinheiten der Formel (VII)

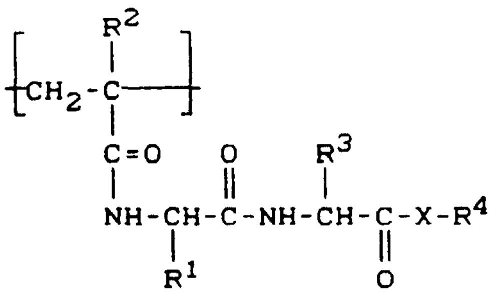

(VII)

enthalten, in der

$R^1$ bis $R^4$ und X die unter Formel (I) angegebene Bedeutung haben.

Vorzugsweise liegen die erfindungsgemäßen optisch aktiven Polymere der Formel (VII) in Form von vernetzten unlöslichen aber quellbaren Perlpolymerisaten oder in an feinteilige anorganische Trägermaterialien wie z.B. Kieselgel gebundener Form vor. Sie können auch als lineare, in geeigneten organischen Lösungsmitteln lösliche Polymere hergestellt werden. Es ist ferner möglich, verschiedene erfindungsgemäße Dipeptide der Formel (I) cozupolymerisieren, sowie 0,1 bis 60, vorzugsweise 0,1 bis 20 Mol-% copolymerisierbarer, anderer Monomere in die Polymere einzubauen.

Die mechanischen Eigenschaften der optisch aktiven Perlpolymerisate, insbesondere die Druckstabilität im gequollenen Zustand können durch Einbau von 2 bis 60 Gew.-X (bezogen auf Monomer und Vernetzer) eines feinteiligen anorganischen Füllstoffs verbessert werden.

Als anorganischer Füllstoff sind unlösliche, feinteilige, kristalline oder amorphe anorganische Verbindungen mit einer mittleren Teilchengröße von 3 nm bis 10 µm, vorzugsweise 10 bis 500 nm geeignet.

Beispiele für geeignete Füllstoffe sind Hydroxide, Oxide, Carbonate, Sulfate und Phosphate von Metallen, wie Aluminiumhydroxid, Aluminiumoxid, Aluminiumoxidhydrat, Titandioxid, Zirkondioxid, Calciumcarbonat, Dolomit, Calciumsulfat, Bariumsulfat, Calciumphosphat und Zirkonphosphat. Bevorzugt sind silikatische Füllstoffe wie beispielsweise Kaolin, calciniertes Kaolin, Glimmer, Wollastonit, Calciumsilikat, Aluminiumsilikat, Natriumaluminiumsilikat, Zirkonsilikat, Quarzmehl und amorphes Siliciumdioxid, desweiteren fein gemahlene Gläser und Glaskeramiken. Besonders bevorzugt ist flammhydrolytisch gewonnenes, mikrofeines Siliciumdioxid, das z.B. unter der Bezeichnung Aerosil oder HDK (Hochdisperse Kieselsäure) als Handelsprodukt erhältlich ist.

Die Größe der spezifischen Oberfläche des anorganischen Füllstoffs ist von Wichtigkeit für die Qualität und die Trenneigenschaften der erfindungsgemäßen Perlpolymerisate. Die spezifische Oberfläche soll 30 bis 500 $m^2$/g, vorzugsweise 50 bis 400 $m^2$ /g, gemessen nach der BET-Methode (Gasadsorption) betragen.

Die anorganischen Füllstoffe gelangen vorzugsweise in mit Haftvermittlern behandelter Form zum Einsatz. Als Haftvermittler eignen sich beispielsweise Silan- und Titanverbindungen, wie Trimethylchlorsilan, Hexamethylendisiloxan, 3-Aminopropyltrimethoxysilan, Butyltitanat und Isopropyltitanat. Besonders bevorzugt sind Haftvermittler mit polymerisierbaren Gruppen wie Vinyltrimethoxysilan, Vinyltriethoxysilan, Vinylethyldiethoxysilan, Allyltriethoxysilan, γ-MethacryloxypropylAllyltriethoxysilan, γ-Methacryloxypropyltrimethoxysilan, γ-Acryloxypropyltrimethoxysilan, γ-Acryloxypropyltriethoxysilan, γ-Methacryloxypropylmethyldiethoxysilan.

Die vernetzten Polymerisate liegen vorzugsweise in Form kleiner Teilchen (Perlen) mit 5 bis 200 µm Teilchendurchmesser vor. Sie werden z.B. durch Suspensionspolymerisation der optisch aktiven Dipeptide der Formel (I) mit 0,5 bis 50 Mol-%, vorzugsweise 1 bis 30 Mol-%, besonders bevorzugt 3 bis 20 Mol-% (bezogen auf die Gesamtmenge [Mol] der eingesetzten Monomere) eines geeigneten Vernetzers in an sich bekannter Weise hergestellt.

Durch die Art und Menge der Vernetzer und des Lösungsmittels läßt sich der Quellungsgrad der (Perl)Polymerisate nach üblichen Methoden einstellen.

Bei der praktischen Verwendung haben sich (Perl)Polymerisate mit einem Quellungsgrad (Q) von 1.1 bis 12.0, bevorzugt von 2.0 bis 6.0 bewährt.

Der Quellungsgrad Q wird wie folgt bestimmt:

$$Q = \frac{\text{Polymerisatvolumen (gequollen)}}{\text{Polymerisatvolumen (ungequollen)}}$$

Als Vernetzungsmittel kommen Verbindungen in Frage, die mindestens zwei polymerisierbare Vinylgruppen enthalten. Bevorzugte Vernetzungsmittel sind Alkandioldiacrylate wie 1,6-Hexandioldiacrylat, 1,4-Butandioldiacrylat,

1,3-Propandioldiacrylat oder 1,2-Ethylenglykoldiacrylat, oder Alkandioldimethacrylate wie 1,4-, 1,3- oder 2,3-Butandioldimethacrylat, 1,3-Propandioldimethacrylat oder 1,2-Ethylenglykoldimethacrylat, aromatische Divinylverbindungen wie beispielsweise Divinylbenzol, Divinylchlorbenzol oder Divinyltoluol, Dicarbonsäuredivinylester wie Adipinsäuredivinylester, Benzoldicarbonsäuredivinylester, Terephthalsäuredivinylester, N,N'-Alkylendiacrylamide wie N,N'-Methylendiacrylamid, N,N'-Ethylendiacrylamid, N,N'-Methylendimethylacrylamid, N,N'-Ethylendimethacrylamid oder N,N'-Dimethyl-ethylendiacrylamid. Ebenso kann N,N',N"-Tris(Acryloyl)-1,3,5-perhydrotriazin oder N,N'-Bis(acryloyl)piperazin verwendet werden.

Als Radikalbildner kommen die üblichen Radikalbildner in Frage. Bevorzugt sind Peroxide wie beispielsweise Dibenzoylperoxid, Dilauroylperoxid oder Di-orthotolylperoxid, Perester wie tert.-Butylperpivalat oder tert.-Butylperoctanoat, oder Azoverbindungen wie beispielsweise Azobisisobuttersäurenitril (AIBN). Auch Gemische verschiedener Radikalbildner sind verwendbar.

Die Polymerisationskomponenten werden in einem nicht mit Wasser mischbaren organischen Lösungsmittel, vorzugsweise einem aliphatischen oder aromatischen Kohlenwasserstoff wie Hexan, Heptan, Isodecan, Benzol oder Toluol, einem Halogenkohlenwasserstoff wie Di-, Tri-, Tetrachlormethan oder 1,2-Dichlorethan, einem Ester wie Essigsäureethylester, Essigsäurebutylester oder Dialkylcarbonaten oder einem nicht wasserlöslichen Keton wie Methylisobutylketon oder Cyclohexanon gelöst.

Die organische Phase wird mit Hilfe eines wirksamen Rührers in der wäßrigen Lösung eines Schutzkolloids, bevorzugt in einer wäßrigen Lösung von Polyvinylalkohol, Polyvinylpyrrolidon oder eines Copolymers aus Methacrylsäure und Methylmethacrylat, gleichmäßig verteilt. Je Gewichtsteil organische Phase verwendet man etwa 1 bis 20, bevorzugt 2 bis 10 Gew.-Teile wäßrige Phase. Das Polymerisationsgemisch wird unter Rühren in einer Inertgasatmosphäre, vorzugsweise unter Stickstoff, auf Temperaturen von 30°C bis 100°C, vorzugsweise 40°C bis 80°C erhitzt. Die Polymerisationsdauer beträgt zwischen 2 und 24, bevorzugt 4 und 12 Stunden. Das auf diese Weise erhaltene Copolymerisat wird durch Filtration von der flüssigen Phase getrennt, durch gründliches Waschen mit Wasser und mit organischen Lösungsmitteln wie Methanol, Ethanol, Benzol, Toluol, Di-, Trichlormethan oder Aceton gereinigt und anschließend getrocknet.

Insbesondere für analytische Anwendungen werden die erfindungsgemäßen optisch aktiven Polymere vorzugsweise in an feinteilige anorganische Träger gebundener Form eingesetzt. Die Herstellung solcher optisch aktiver Chromatographiephasen kann beispielsweise nach den in DE-A 3 706 890 beschriebenen Verfahren erfolgen.

Bevorzugt ist die Polymerisation der optisch aktiven Dipeptide der Formel (I) in Gegenwart von Kieselgel-Vinylphasen, die nach bekannten Methoden erhältlich sind, oder von Kieselgel-Methacrylatphasen, die durch Veresterung von Kieselgel-Diolphasen oder durch Umsetzung von Kieselgel mit γ-Methacryloxypropyl-trimethoxysilan erhalten werden können. Die Polymerisation kann dabei in Abwesenheit von Lösungsmitteln oder in Gegenwart von Lösungsmitteln oder von Fällungsmitteln für die Polymeren aus den Dipeptiden der Formel (I) durchgeführt werden. Als Initiatoren können die zur Herstellung der Perlpolymerisate verwendeten Radikalbildner ebenfalls eingesetzt werden.

Die polymermodifizierten Kieselgele enthalten vorzugsweise 1 bis 40 Gew.-%, insbesondere 3 bis 30 Gew.-% an optisch aktivem Monomer (I), bezogen auf das Gesamtgewicht. Sie werden intensiv mit Lösungsmitteln für das Polymere gewaschen und im Vakuum getrocknet.

Selbstverständlich können auch hier Gemische von zwei oder mehreren der erfindungsgemäßen Dipeptide gegebenenfalls auch mit weiteren copolymerisierbaren Monomeren eingesetzt werden.

Die Erfindung betrifft ferner die Verwendung der erfindungsgemäßen Polymere der Formel (I) als solche oder in vernetzter bzw. an Kieselgel gebundener Form zur chromatographischen Trennung von racemischen Gemischen in die optischen Antipoden. Besonders bewährt haben sich die erfindungsgemäßen Polymerisate zur chromatographischen Trennung von Hexahydrocarbazol-Derivaten wie z,B. 3-Γ-(4-Fluorphenylsulfonamido)-9-(2-carboxyethyl)-1,2,3,4,4a,9a-hexahydrocarbazol (Rac I), Benzodiazepinen wie z.B. Oxazepam (Rac II), Arylpropionsäuren wie z.B. 2-(4-Isobutylphenyl)-propansäure (Rac III), 2-(3-Benzoyl)-propansäure (Rac IV), Dihydropyridin-Derivaten wie z,B. 1,4-Dihydro-2,6-dimethyl-4-(2-trifluormethylphenyl)-pyridin-3,5-dicarbonsäure-5-methylester (Rac V), 1,4-Dihydro-2,6-dimethyl-4-(3-nitrophenyl)-3,5-dicarbonsäure-3-isopropyl-5-(2-methoxyethyl)-ester (Rac VI), Chlorthalidon (Rac VII), Biaryle wie z.B. 2,2'-Dimethoxy-6,6'-dinitro-biphenyl (Rac VIII), Cyclopropancarbonsäuren wie z.B. cis-3-[2-Chlor-4-(2-chlorphenyl)ethenyl]-2,2-dimethylcyclopropancarbonsäure (Rac IX). Die Zusammensetzung des Fließmittels kann je nach Art und Eigenschaft des zu trennenden Racemates in üblicher Weise ausgewählt und optimiert werden. Die an Kieselgel gebundenen erfindungsgemäßen Polymere können für chromatographische Racemattrennungen unter HPLC-Bedingungen eingesetzt werden.

Die Fähigkeit der Polymerisate zur Racemattrennung wird durch die Kapazitätsverhältnisse ($k'_{1(2)}$-Werte) für die beiden Enantiomere (1) und (2) und den daraus resultierenden Enantioselektivitätswert α ausgedrückt. Diese chromatographischen Parameter sind wie folgt definiert:

$$\text{Kapazitätsverhältnis } k'_{1(2)} = \frac{t_{1(2)} - t_o}{t_o}$$

$$\text{Enantioselektivität } \alpha = \frac{k'_2}{k'_1}$$

$t_o$ = Totzeit der Säule

$t_{1(2)}$ = Retentionszeit des zuerst eluierten Enantiomers 1 bzw. des später eluierten Enantiomers 2

Die präparative Trennung von racemischen Gemischen in ihre optischen Antipoden unter Verwendung der erfindungagemäßen Polymerisate wird vorzugsweise säulenchromatographisch vorgenommen. Besonders vorteilhaft ist es hierfür, die chromatographische Trennung mit Perlpolymerisaten einer bestimmten Korngrößenverteilung vorzunehmen; gute Trennleistungen werden mit Perlpolymerisaten einer Korngrößenverteilung von 5 bis 200 µm, bevorzugt 15 bis 100 µm erhalten.

Die Arbeitsmethodik der säulenchromatographischen Trennung ist bekannt. Üblicherweise wird das Polymerisat in Fließmittel suspendiert und die Suspension in eine Glassäule gefüllt. Nach Ablaufen des Fließmittels wird das zu trennende Racemat, gelöst im Fließmittel, auf die Säule aufgebracht. Dann wird mit Fließmittel eluiert und die Enantiomeren im Eluat photometrisch und/oder polarimetrisch mittels geeigneter Durchflußzellen detektiert.

Als Fließmittel werden üblicherweise organische Lösungsmittel bzw. Lösungsmittelgemische verwendet, die das als Adsorbens eingesetzte Polymerisat anquellen und das zu trennende Racemat lösen. Beispielsweise seien genannt: Kohlenwasserstoffe wie Benzol, Toluol oder Xylol, Ether wie Diethylether, tert.-Butylmethylether, Dioxan oder Tetrahydrofuran, Halogenkohlenwasserstoffe wie Di- oder Trichlormethan, Aceton, Acetonitril oder Essigester, Alkohole wie Methanol, Ethanol, n-Propanol, Isopropanol oder n-Butanol, oder aber Gemische der genannten Lösungsmittel. Als besonders geeignet haben sich Mischungen aus Toluol mit Tetrahydrofuran, Dioxan oder Isopropanol erwiesen.

Die überraschend guten Trenneigenschaften der erfindungsgemäßen Trennphasen seien an einigen Beispielen erläutert. Die Trennphasen werden jeweils nach dem gleichen Verfahren hergestellt und in an Kieselgel gebundener Form eingesetzt.

Tabelle 1 zeigt einen Vergleich der Enantioselektivitäten α von Trennphasen mit strukturell gleicher "Endgruppe"; auf der einen Seite die einer erfindungsgemäßen N-Methacryloyl-S-Leu-S-Ile-t-butylester-Phase A, auf der anderen Seite die einer N-Methacryloyl-S-Ile-t-butylester-Phase B, wodurch der erfindungsgemäße Unterschied beim Übergang von einer Aminosäure-Phase B zu einer Dipeptid-Phase A offensichtlich wird.

Tabelle 1

| Racemat | α an Dipeptid-Phase A | α an Aminosäure-Phase B |
|---|---|---|
| Rac I | 16,70 | 3,11 |
| Rac II | 2,00 | <1,02 |
| Rac V | 1,35 | <1,02 |
| Rac VII | 2,20 | 1,16 |
| Rac VIII | 12,00 | 1,35 |
| Rac IX | 1,53 | <1,02 |

Tabelle 2 zeigt einen Vergleich der Enanticselektivitäten α von Trennphasen mit strukturell gleicher "Kopfgruppe"; auf der einen Seite die einer erfindungsgemäßen N-Methacryloyl-S-Nle-S-Ile-t-butylester-Phase C, auf der anderen Seite die einer N-Methacryloyl-S-Nle-d-menthylamid-Phase D, wodurch ebenfalls der überraschende Unterschied zwischen einer erfindungsgemäßen Dipeptid-Phase C und einer "einfachen" Aminosäure-Phase D erkennbar wird.

Tabelle 2

| Racemat | α an Dipeptid-Phase C | α an Aminosäure-Phase D |
|---|---|---|
| Rac III | 1,16 | <1,02 |
| Rac IV | 1,10 | <1,02 |
| Rac VI | 1,10 | <1,02 |
| Rac VIII | 8,75 | 3,20 |

Tabelle 2 (fortgesetzt)

| Racemat | α an Dipeptid-Phase C | α an Aminosäure-Phase D |
|---|---|---|
| Rac IX | 1,45 | <1,02 |

Ausführungsbeispiele

Weg A: Edukte N-BOC-Aminosäuren

Beispiel 1: N-Methacryloyl-S-Val-S-Ala-1-bornylester

Man legte 21,7 g N-BOC-S-Val in 250 ml Tetrahydrofuran vor, tropfte bei 0°C 10,1 g Triethylamin zu und bei -13°C 10,9 g Chlorameisensäureethylester. Nach 10 Minuten bei dieser Temperatur wurden 22,5 g S-Ala-1-bornylester in 75 ml Tetrahydrofuran zugetropft. Man ließ auftauen, rotierte ein, nahm in Dichlormethan auf und wusch mit Wasser, 1 N HCl sowie halbgesättigter Natriumhydrogencarbonat-Lösung und trocknete über Magnesiumsulfat. Es wurde auf 100 ml einrotiert, bei 0°C mit 100 ml Trifluoressigsäure versetzt und 2 Stunden bei Raumtemperatur nachgerührt. Es wurde eingeengt, in Dichlormethan aufgenommen und unter Eiskühlung mit 20 %iger Natronlauge auf pH 11-12 gebracht. Man extrahierte noch 2 x mit Dichlormethan, trocknete über Magnesiumsulfat und engte auf ein Volumen von 275 ml ein. Bei 0°C fügte man 11,1 g N-Methylmorpholin und 9,6 g Methacryloylchlorid in 50 ml Dichlormethan zu, rührte 1 Stunde bei Raumtemperatur nach, wusch mit Wasser, 1 N HCl und halbgesättigter Natriumhydrogencarbonat-Lösung und trocknete die organischen Extrakte über $MgSO_4$. Das erhaltene Rohprodukt (31,6 g) reinigte man chromatographisch an Kieselgel mit t-Butylmethylether/Petrolether und erhielt 19,5 g Produkt vom Fp. 35°C.

Beispiel 2: N-Methacryloyl-S-Val-S-Val-t-butylamid

Zu einer Lösung von 3,14 g N-BOC-S-Val-hydroxysuccinimidester in 250 ml Diethylether tropfte man 2 g S-Val-L-butylamid und 2 g N-Methylmorpholin in 200 ml des gleichen Lösungsmittels. Man rührte über Nacht nach, wusch 2 x mit Wasser, trocknete über Magnesiumsulfat und engte im Vakuum zur Trockene ein. Nach Kristallisation aus t-Butylmethylether/Petrolether erhielt man 3 g Produkt vom Fp. 230°C.

Weg B: Edukte N-(Meth)Acryloyl-aminosäuren

Beispiel 3: N-Methacryloyl-S-Ala-S-Ala-1-bornylester

Zu einer Lösung von 15,7 g N-Methacryloyl-S-Ala in 100 ml Dichlormethan tropfte man bei 0°C 22,5 g S-Ala-1-bornylester in 250 ml Dichlormethan und anschließend 27,2 g 1-Ethoxycarbonyl-2-ethoxy-1,2-dihydrochinolin (EEDQ) in 125 ml Dichlormethan. Man rührte 3 Tage bei Raumtemperatur nach, wusch 5 x mit 1 N HCl, trocknete über Magnesiumsulfat und engte zur Trockene ein. Nach Kristallisation des Rohprodukts (37 g) aus Heptan erhielt man 20,6 g Titelverbindung vom Fp. 60-62°C.

Beispiel 4: N-Acryloyl-S-Phe-S-Phe-isopropylester

Zu 21,9 g N-Acry oyl-S-phenylalanin in 400 ml Tetrahydrofuran tropfte man bei 0°C zunächst 20,7 g S-Pheisopropylester in 50 ml Tetrahydrofuran und anschließend eine Lösung von 27,3 g EEDQ in 100 ml abs. THF. Man rührte 3 Tage bei Raumtemperatur nach, engte zur Trockene ein und nahm den Rückstand in Dichlormethan auf. Man wusch 5 x mit 1 N HCl, trocknete über Magnesiumsulfat und rotierte am Rotationsverdampfer ein. Man erhielt 54 g Rohprodukt und nach Kristallisation aus Toluol/Petrolether 33,7 g Produkt vom Fp, 134°C.

Beispiel 5: N-Methacryloyl-S-Leu-S-Phe-d-menthylester

Zu einer Lösung von 19,9 g N-Methacryloyl-S-Leu in 200 ml Essigester tropfte man bei 0°C 30,3 g S-Phe-d-menthylester in 100 ml Essigester und anschließend 27,2 g EEDQ in 100 ml des gleichen Lösungsmittels. Es wurde 3 Tage bei Raumtemperatur nachgerührt und wie oben beschrieben aufgearbeitet. Durch chromatographische Reinigung des Rohprodukts an Kieselgel mit t-Butylmethylether/Petrolether erhielt man 20,2 g Titelverbindung vom Fp. 38°C.

Tabelle 3: Monomere Dipeptide der Formel (I)

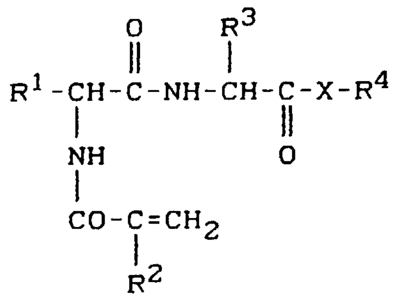

(I)

| Bsp. Nr. | $R^1$ | $R^2$ | $R^3$ | X | $R^4$ | Fp. (°C) | Drehwert $\alpha_D$ (C=1, $CHCl_3$) | Name |
|---|---|---|---|---|---|---|---|---|
| 6 | $CH_3$ | $CH_3$ | $CH_2SCH_3$ | NEt | Et | 110 | -53,7 | N-MA*-S-Ala-R-(S)-methyl-Cys-diethylamid |
| 7 | $CH_3$ | $CH_3$ | $(CH_2)_2SCH_3$ | NH | 3-Pentyl | 177-79 | -54,9 | N-MA-S-Ala-S-Met-3-pentylamid |
| 8 | $CH_3$ | $CH_3$ | iPr | O | t-Butyl | 105 | | N-MA-S-Ala-S-Val-t-butylester |
| 9 | $CH_3$ | $CH_3$ | sBu | O | t-Butyl | 60-65 | -13,0 | N-MA-S-Ala-S-Ile-t-butylester |
| 10 | Ph | $CH_3$ | iPr | O | t-Butyl | 156 | +73,3° | N-MA-S-Phg-S-Val-t-butylester |

* MA = Methacryloyl
A = Acryloyl

Tabelle 3 - Fortsetzung

| Bsp. Nr. | $R^1$ | $R^2$ | $R^3$ | X | $R^4$ | Fp. (°C) | Drehwert $\alpha_D$ (C=1, $CHCl_3$) | Name |
|---|---|---|---|---|---|---|---|---|
| 11 | $CH_3$ | $CH_3$ | iBu | O | d-Menthyl | 155 | | N-MA*-S-Ala-S-Leu-d-menthylester |
| 12 | $CH_3$ | $CH_3$ | $CH_2Ph$ | O | d-Menthyl | Öl | | N-MA-S-Ala-S-Phe-d-menthylester |
| 13 | nBu | $CH_3$ | sBu | O | t-Butyl | 136 | -9,3 | N-MA-S-Nle-S-Ile-t-butylester |
| 14 | iBu | $CH_3$ | $CH_2Ph$ | O | l-Menthyl | 104 | | N-MA-S-Leu-R-Phe-l-menthylester |
| 15 | iBu | $CH_3$ | sBu | O | t-Butyl | 174 | -97,7 | N-Ma-S-Leu-S-Ile-t-butylester |
| 16 | i-Bu | $CH_3$ | iBu | O | d-Menthyl | Öl | | N-MA-S-Leu-S-Leu-d-menthylester |
| 17 | $(CH_2)_2SCH_3$ | $CH_3$ | $CH_2Ph$ | O | d-Menthyl | 103 | +25,2 | N-MA-S-Met-S-Phe-d-menthylester |
| 18 | Ph | $CH_3$ | iPr | O | t-Butyl | 123-24 | -52,2 | N-MA-R-Phg-S-Val-t-butylester |
| 19 | iBu | $CH_3$ | sBu | O | 3-Pentyl | 132 | -31,6 | N-MA-S-Leu-S-Ile-3-pentylester |
| 20 | Ph | $CH_3$ | $(CH_2)_2SCH_3$ | NEt | Et | 167 | +51,9 | N-Ma-S-Phg-S-Met-diethylamid |
| 21 | iBu | $CH_3$ | Cyclohexyl | NEt | Et | 99 | -10,8 | N-Ma-S-Leu-R-hexahydrophenylglycin-diethylamid |

* MA = Methacryloyl
A = Acryloyl

Polymerisation der optisch aktiven Dipeptide der Formel I auf Kieselgel

a) Herstellung von Vinylsilica

Unter strengem Feuchtigkeitsausschluß werden 50 g bei 120°C getrocknetes Kieselgel (LiChrosorb Si 100, 5 µ, Merck; Polygosil 100-5, Macherey & Nagel) mit 600 ml trockenem Toluol versetzt. 100 ml Toluol werden bei Normaldruck unter $N_2$ abdestilliert.

Nach Abkühlen auf 30°C werden 35 g Trichlorvinylsilan zugegeben. Der Ansatz wird unter Rühren zum Rückfluß erhitzt. Dann werden 62 g Triethylamin in 150 ml Toluol innerhalb einer Stunde zugetropft. Man läßt über Nacht bei 105-108°C weiterrühren.

Nach Abkühlen und Absaugen über eine Glasfritte wird das Kieselgel nacheinander in Toluol, Dichlormethan, Methanol, Methanol/Wasser = 60/40, zweimal Methanol und zweimal Dichlormethan ausgerührt, zwischendurch jeweils gründlich trockengesaugt und am Ende bei 70°C getrocknet.

Analysen: C: 2,8-3,2%; H:0,7-0,9%

b) Herstellung der Verbindungen der Formel VII in an Kieselgel gebundener Form

Beispiel 20

3,0 g Vinylsilica, 2,0 g Monomer (siehe Tabelle 4) und 40 mg Azobisisobutyronitril werden in 8 ml Toluol, Toluol/Heptan-Gemischen oder Chloroform gelöst bzw. suspendiert. Unter Magnetrührung wird die Apparatur dreimal evakuiert und mit Stickstoff belüftet. Die Mischung wird 1 Stunde bei Raumtemperatur gerührt, dann rasch auf 80°C (bzw. 65°C für Chloroform) erwärmt und 1 Std. bzw. 4 Stunden bei Chloroformansätzen bei dieser Temperatur belassen. Nach Zusatz von 100 mg 2,6-Di-tert.-butyl-4-methylphenol wird rasch auf Raumtemperatur gekühlt. Das Kieselgel wird über eine G4-Fritte abgesaugt, zweimal in Chloroform, einmal in Toluol und einmal in Isopropanol je 15 min ausgerührt, gut trockengesaugt und bei Raumtemperatur im Vakuum (<0,005 atm) getrocknet.
Ausbeute: 3,35 g ± 10%
Belegungsgrad siehe Tabelle 4.

Tabelle 4:

| Kieselgelphasen | | | | | |
|---|---|---|---|---|---|
| Beispiel Nr. | Monomer Nr./g | | Lösungsmittel | N-Gehalt [%] | gebundenes Polymer [Gew.-%] |
| 21 | 17 | 2,0 | Toluol | 1,1 | 19,8 |
| 22 | 7 | 1,0 | $CHCl_3$ | 1,3 | 11,1 |
| 23 | 6 | 1,0 | Toluol | 2,8 | 22,0 |
| 24 | 19 | 2,0 | Toluol | 1,15 | 15,7 |
| 25 | 4 | 2,0 | $CHCl_3$ | 0,7 | 10,2 |
| 26 | 5 | 1,5 | Toluol | 0,6 | 10,4 |
| 27 | 12 | 2,0 | Toluol | 1,05 | 16,6 |
| 28 | 14 | 2,0 | Toluol | 0,75 | 13,0 |
| 29 | 15 | 2,0 | Toluol | 1,35 | 17,8 |
| 30 | 2 | 2,0 | $CHCl_3$ | 1,2 | 9,7 |
| 31 | 3 | 2,0 | Toluol | 1,5 | 19,6 |
| 32 | 13 | 2,0 | Toluol | 0,9 | 11,9 |
| 33 | 16 | 2,0 | Toluol | 0,75 | 12,1 |

c) Polymerisation der optisch aktiven Dipeptide der Formel I zu Perlpolymerisaten gemäß Formel VII

Beispiel 34

In eine 250 ml-Rührapparatur werden 13,0 g N-Methacryloyl-S-Ala-S-Leu-d-menthylester (vgl. Monomerbeispiel 12)), 2,0 g Ethylenglykoldimethacrylat (EGDMA), 0,5 g Azobisisobutyronitril, 37,5 g Trichlormethan und eine Lösung von 4 g Polyvinylalkohol in 130 ml 1M Phosphatpuffer pH 6,0 eingefüllt. Die Apparatur wird mehrmals evakuiert und mit Stickstoff wieder befüllt. Die Mischung wird dann unter Stickstoff mit 425 U/min gerührt und 15 min bei 50°C und 14 Stunden bei 55°C polymerisiert. Die Feinstanteile <10µm werden durch mehrmaliges Sedimentieren in Wasser abgetrennt, die Perlen abgesaugt, mit Aceton, Trichlormethan und wieder Aceton intensiv gewaschen und bei 50°C getrocknet.

Ausbeute: 12,3 g Perlpolymerisat
Korngröße: 15-50 µm
Schüttvolumen: 1,9 ml/g
Quellvolumen: 5,4 ml/g (in Toluol/THF=3/2 (V/V))

**Patentansprüche**

1. Polymerisierbare optisch aktive Dipeptide der allgemeinen Formel (I)

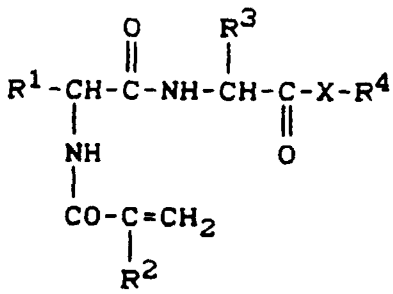

(I)

in der

$R^1$ und $R^3$ gleich oder verschieden sind und für $C_1$-$C_5$-Alkyl, $CH_2$-O-A, $CH_2$-S-A, $(CH_2)_2$-S-$CH_3$, $CH_2$-Cyclohexyl, Cyclohexyl, Phenyl, Benzyl, 4-A-O-Benzyl, $CH_2$-Benzyl, Indolyl, $CH_2$-Naphthyl oder Naphthyl stehen, wobei A Wasserstoff, Methyl, t-Butyl oder Benzyl ist,

$R^2$ Wasserstoff, Methyl oder Fluor bedeutet,

X für Sauerstoff oder $NR^5$ steht,

wobei $R^5$ Wasserstoff, Methyl, Ethyl ist oder zusammen mit $R^4$ einen $C_5$-$C_6$-Cycloalkyl-Rest bildet und $R^4$ für einen geradkettigen oder verzweigten $C_3$-$C_{18}$-Alkyl- oder einen ein- bis vierfach $C_1$-$C_4$-alkyl-substituierten $C_3$-$C_{12}$-Cyloalkylrest, Benzyl, 1-Phenylethyl oder für ein- bis zweifach durch Fluor, Chlor, Trifluormethyl, Methoxy oder $C_1$-$C_4$- alkyl substituiertes Phenyl steht.

2. Polymerisierbare optisch aktive Dipeptide nach Anspruch 1, wobei

$R^1$, $R^3$ und $R^5$ die in Anspruch 1 angegebene Bedeutung haben

$R^2$ Wasserstoff oder Methyl bedeutet und

$R^4$ für $C_3$-$C_8$-Alkyl, $C_5$-$C_7$-Cycloalkyl, bis zu vierfach $C_1$-$C_4$-alkylsubstituiertes $C_6$-Cycloalkyl, Benzyl, 1-Phenylethyl, Phenyl, 4-t-Butylphenyl, 3,5-Dichlorphenyl oder 3,5-Dimethylphenyl steht.

3. Polymerisierbare optisch aktive Dipeptide nach Anspruch 1, die sich von den Aminosäuren Alanin, Aminobuttersäure, Valin, Norvalin, Leucin, Isoleucin, Terleucin, Norleucin, Neopentylglycin, Serin, Cystein, Methionin, Hexahydrophenylalanin, Hexahydrophenylglycin, Phenylglycin, Phenylalanin, Tyrosin, Homophenylalanin, Tryptophan, Naphthylalanin oder Naphthylglycin ableiten.

4. Polymerisierbare optisch aktive Dipeptide nach Anspruch 1, wobei es sich um die Dipeptide N-Methacryloyl-S-Leu-S-Ile-O-t-butylester, N-Methacryloyl-S-Leu-S-Phe-d-menthylester, N-Methacryloyl-S-Leu-S-Leu-3-pentylamid, N-MethacrylaylS-Val-S-Val-t-butylamid, N-Acryloyl-S-Phe-S-Phe-2-propylester, N-Methacryloyl-S-Val-S-Ala-1-bornylester, N-Methacryloyl-S-Ala-S-Met-3-pentylamid, N-Methacryloyl-R-Phg-S-Val-t-butylester, N-Methacryloyl-S-Leu-R-Phe-1-menthylester, N-Methacryloyl-S-Ala-R-(S)-methyl-Cys-diethylamid, N-Methacryloyl-S-Ala-S-Leu-d-menthylester handelt.

5. Verfahren zur Herstellung der polymerisierbaren optisch aktiven Dipeptide nach Anspruch 1, dadurch gekennzeichnet, daß man

A) Dipeptidderivate der Formel (II)

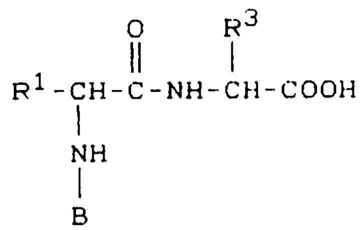

(II)

in welcher

$R^1$ und $R^3$ die oben angegebene Bedeutung haben und

B für eine in der Peptidchemie übliche, leicht abzuspaltende Gruppe steht,

mit in der Peptidchemie üblichen Verknüpfungsreaktionen mit dem Rest $XR^4$ verknüpft wobei X und $R^4$ die oben angegebene Bedeutung haben und anschließend den Rest B mit in der Peptidchemie üblichen Methoden abspaltet und die dann erhaltenen Verbindungen der allgemeinen Formel (III)

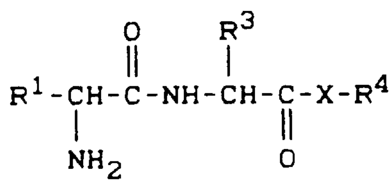

(III)

gegebenenfalls in Form ihrer Säureadditionsprodukte mit Acryloylderivaten der Formel (IV)

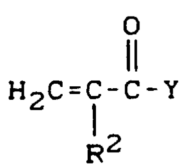

(IV)

in welcher

$R^2$ die oben angegebene Bedeutung hat und Y für Fluor, Chlor, Brom oder für den Rest $-OCO-OR^2=CH_2$ steht,

in Gegenwart eines säurebindenden Mittel in inerten organischen Lösungsmitteln zu Verbindungen der Formel (I) umsetzt, oder

B) Aminosäurederivate der Formel (V)

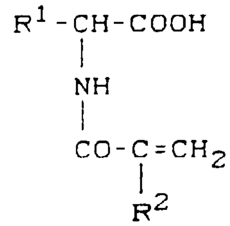

(V)

in welcher

$R^1$ und $R^2$ die oben angegebene Bedeutung haben,

mit einem zweiten Aminosäurederivat der Formel (VI)

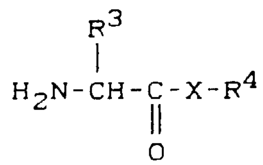

(VI)

in welcher

$R^3$, Y und $R^4$ die oben angegebene Bedeutung haben,

nach üblichen Methoden zu Dipeptiden der Formel (I) verknüpft.

6. Optisch aktive Polymere und Copolymere erhältlich durch Polymerisation bzw. Copolymerisation der optisch aktiven Dipeptide nach Anspruch 1.

7. Optisch aktive Polymere und Copolymere nach Anspruch 6, dadurch gekennzeichnet, daß sie mindestens 40 Mol-% Struktureinheiten der Formel (VII)

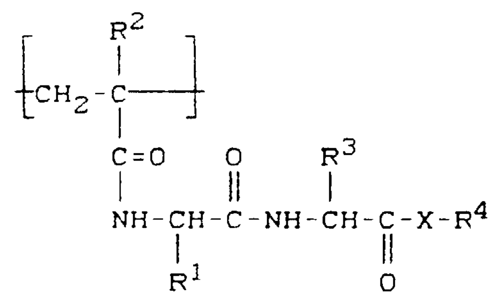

(VII)

enthalten,

8. Verwendung der optisch aktiven Dipeptide nach Anspruch 1 zur Herstellung von optisch aktiven Polymeren und Copolymeren.

9. Verwendung der optisch aktiven Polymere und Copolymere nach Anspruch 6 zur chromatographischen Trennung von racemischen Gemischen in die optischen Antipoden.

10. Verwendung der optisch aktiven Polymere und Copolymere nach Anspruch 6 zur chromatographischen Trennung von von Hexahydrocarbazol-Derivaten wie z.B. 3-r-(4-Fluorphenylsulfonamido)-9-(Z-carboxyethyl)-1,2,3,4,4a,9a-hexahydrocarbazol (Rac I), Benzodiazepinen wie z.B. Oxazepam (Rac II), Arylpropionsäuren wie z.B. 2-(4-Isobutylphenyl)-propansäure (Rac III), 2-(3-Benzoyl)-propansäure (Rac IV), Dihydropyridin-Derivaten wie z.B. 1,4-Dihydro-2,6-dimethyl-4-(2-trifluormethylphenyl)-pyridin-3,5-dicarbonsäure-5-methylester (Rac V), 1,4-Dihydro-2,6-dimethyl-4-(3-nitrophenyl)-3,5-dicarbonsäure-3-isopropyl-5-(2-methoxyethyl)-ester (Rac VI), Chlorthalidon (Rac VII), Biaryle wie z.B. 2,2'-Dimethoxy-6,6'-dinitro-biphenyl (Rac VIII), Cyclopropancarbonsäuren wie z.B, cis-3-[2-Chlor-4-(2-chlorphenyl)ethenyl]-2,2-dimethylcyclopropancarbonsäure (Rac IX).

**Claims**

1. Polymerisable optically active dipeptides of the general formula (I)

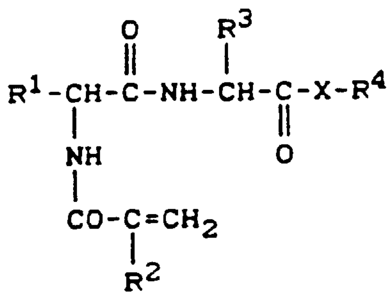

(I)

in which

| | |
|---|---|
| $R^1$ and $R^3$ | are identical or different and represent $C_1$-$C_5$-alkyl, $CH_2$-O-A, $CH_2$-S-A, $(CH_2)_2$-S-$CH_3$, $CH_2$-cyclohexyl, cyclohexyl, phenyl, benzyl, 4-A-O-benzyl, $CH_2$-benzyl, indolyl, $CH_2$-naphthyl or naphthyl, where A is hydrogen, methyl, t-butyl or benzyl, |
| $R^2$ | denotes hydrogen, methyl or fluorine, |
| X | represents oxygen or $NR^5$, |

where $R^5$ is hydrogen, methyl, ethyl or, together with $R^4$, forms a $C_5$-$C_6$-cycloalkyl radical, and $R^4$ represents a straight-chain or branched $C_3$-$C_{18}$-alkyl radical or a mono- to tetra-$C_1$-$C_4$-alkyl-substituted $C_3$-$C_{12}$-cycloalkyl radical, benzyl, 1-phenylethyl or represents a phenyl which is mono- to disubstituted by fluorine, chlorine, trifluoromethyl, methoxy or $C_1$-$C_4$-alkyl.

2. Polymerisable optically active dipeptides according to Claim 1, where

| | |
|---|---|
| $R^1$, $R^3$ and $R^5$ | have the meaning stated in Claim 1, |
| $R^2$ | denotes hydrogen or methyl, and |
| $R^4$ | represents $C_3$-$C_8$-alkyl, $C_5$-$C_7$-cycloalkyl, up to tetra-$C_1$-$C_4$-alkyl-substituted $C_6$-cycloalkyl, benzyl, 1-phenylethyl, phenyl, 4-t-butylphenyl, 3,5-dichlorophenyl or 3,5-dimethylphenyl. |

3. Polymerisable optically active dipeptides according to Claim 1, which are derived from the amino acids alanine, amino butyric acid, valine, norvaline, leucine, isoleucine, terleucine, norleucine, neopentylglycine, serine, cysteine, methionine, hexahydrophenylalanine, hexahydrophenylglycine, phenylglycine, phenylalanine, tyrosine, homophenylalanine, tryptophan, naphthylalanine or naphthylglycine.

4. Polymerisable optically active dipeptides according to Claim 1, where the dipeptides are N-methacryloyl-S-Leu-S-Ile O-t-butyl ester, N-methacryloyl-S-Leu-S-Phe d-menthyl ester, N-methacryloyl-S-Leu-S-Leu 3-pentylamide, N-methacryloyl-S-Val-S-Val t-butylamide, N-acryloyl-S-Phe-S-Phe 2-propyl ester, N-methacryloyl-S-Val-S-Ala 1-bornyl ester, N-methacryloyl-S-Ala-S-Met 3-pentylamide, N-methacryloyl-R-Phg-S-Val t-butyl ester, N-methacryloyl-S-Leu-R-Phe 1-menthyl ester, N-methacryloyl-S-Ala-R-(S)-methyl-Cys diethylamide, N-methacryloyl-S-Ala-S-Leu d-menthyl ester.

**5.** Process for the preparation of the polymerisable optically active dipeptides according to Claim 1, characterised in that

A) dipeptide derivatives of the formula (II)

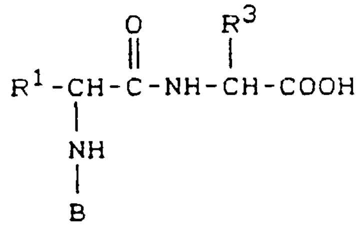

(II)

in which

$R^1$ and $R^3$ have the abovementioned meaning, and

B represents a group which is customary in peptide chemistry and is easy to eliminate,

are linked using the linking reactions customary in peptide chemistry to the radical $XR^4$
where X and $R^4$ have the abovementioned meaning,
and subsequently the radical B is eliminated using the methods customary in peptide chemistry, and the compounds which are then obtained, of the general formula (III)

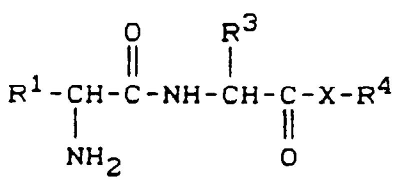

(III)

are reacted, where appropriate in the form of their acid addition products, with acryloyl derivatives of the formula (IV)

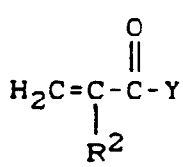

(IV)

in which

$R^2$ has the abovementioned meaning, and

Y represents fluorine, chlorine, bromine or represents the radical $-OCO-CR^2=CH_2$,
in the presence of an acid-binding agent in inert organic solvents to give compounds of the formula (I), or

B) amino acid derivatives of the formula (V)

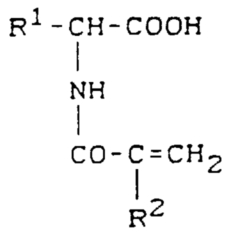

(V)

in which

$R^1$ and $R^2$ have the abovementioned meaning, are linked to a second amino acid derivative of the formula (VI)

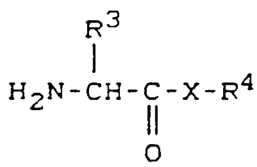

(VI)

in which

$R^3$, Y and $R^4$ have the abovementioned meaning,

by customary methods to give dipeptides of the formula (I).

**6.** Optically active polymers and copolymers obtainable by polymerisation and copolymerisation, respectively, of the optically active dipeptides according to Claim 1.

**7.** Optically active polymers and copolymers according to Claim 6, characterised in that they contain at least 40 mol % of structural units of the formula (VII)

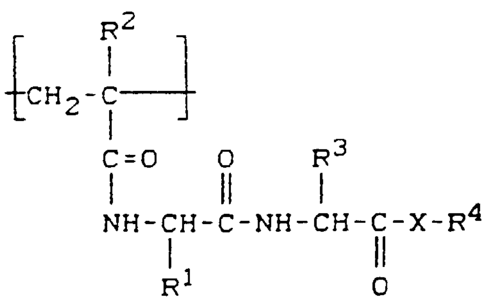

(VII)

**8.** Use of the optically active dipeptides according to Claim 1 for the preparation of optically active polymers and copolymers.

**9.** Use of the optically active polymers and copolymers according to Claim 6 for the chromatographic separation of racemic mixtures into the optical antipodes.

**10.** Use of the optically active polymers and copolymers according to Claim 6 for the chromatographic separation of hexahydrocarbazole derivatives such as for example 3-r-(4-fluorophenylsulphonamido)-9-(2-carboxyethyl)-1,2,3,4,4a,9a-hexahydrocarbazole (Rac I), benzodiazepines such as, for example, oxazepam (Rac II), arylpropionic acids such as, for example, 2-(4-isobutylphenyl)propanoic acid (Rac III), 2-(3-benzoyl)propanoic acid (Rac IV), dihydropyridine derivatives, such as, for example, 1,4-dihydro-2,6-dimethyl-4-(2-trifluoromethylphenyl)pyridine-3,5-dicarboxylic acid 5-methyl ester (Rac V) , 3-isopropyl 5-(2-methoxyethyl) 1,4-dihydro-2,6-dimethyl-4-(3-nitrophenyl)-3,5-dicarboxylate (Rac VI), chlorthalidone (Rac VII), biaryls such as, for example, 2,2'-dimethoxy-6,6'-dinitrobiphenyl (Rac VIII), cyclopropanecarboxylic acids such as, for example, cis-3-[2-chloro-4-(2-chlorophenyl) ethenyl]-2,2-dimethylcyclopropanecarboxylic acid (Rac IX).

## Revendications

**1.** Dipeptides optiquement actifs polymérisables répondant à la formule générale (I)

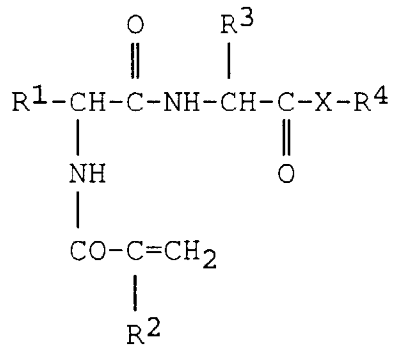

(I)

dans laquelle

- $R^1$ et $R^3$ sont identiques ou différents et représentent un groupe alkyle en $C_1$-$C_5$, un groupe $CH_2$-O-A, un groupe $CH_2$-S-A, un groupe $(CH_2)_2$-S-$CH_3$, un groupe $CH_2$-cyclohexyle, un groupe cyclohexyle, un groupe phényle, un groupe benzyle, un groupe 4-A-O-benzyle, un groupe $CH_2$-benzyle, un groupe indolyle, un groupe $CH_2$-naphtyle ou un groupe naphtyle, A représentant un atome d'hydrogène, un groupe méthyle, un groupe t-butyle ou un groupe benzyle,
- $R^2$ représente un atome d'hydrogène, un groupe méthyle ou un atome de fluor,
- X représente un atome d'oxygène ou un groupe $NR^5$, où $R^5$ représente un atome d'hydrogène, un groupe méthyle, un groupe éthyle ou forme, conjointement avec $R^4$, un radical cycloalkyle en $C_5$-$C_6$, et
- $R^4$ représente un groupe alkyle en $C_3$-$C_{18}$ à chaîne droite ou ramifiée ou encore un radical cycloalkyle en $C_3$-$C_{12}$ portant de 1 à 4 substituants alkyle en $C_1$-$C_4$ identiques ou différents, un groupe benzyle, un groupe 1-phényléthyle ou encore un groupe phényle portant de un à deux substituants identiques ou différents fluoro, chloro, trifluorométhyle, méthoxy ou alkyle en $C_1$-$C_4$.

**2.** Dipeptides optiquement actifs polymérisables selon la revendication 1, dans lesquels

- $R^1$, $R^3$ et $R^5$ ont la signification indiquée à la revendication 1,
- $R^2$ représente un atome d'hydrogène ou un groupe méthyle et
- $R^4$ représente un groupe alkyle en $C_3$-$C_8$, un groupe cycloalkyle en $C_5$-$C_7$, un groupe cycloalkyle en $C_6$ portant jusqu'à 4 substituants alkyle en $C_1$-$C_4$ identiques ou différents, un groupe benzyle, un groupe 1-phényléthyle, un groupe phényle, un groupe 4-t-butylphényle, un groupe 3,5-dichlorophényle ou un groupe 3,5-diméthylphényle.

**3.** Dipeptides optiquement actifs polymérisables selon la revendication 1, qui dérivent des aminoacides choisis parmi le groupe comprenant l'alanine, l'acide aminobutyrique, la valine, la norvaline, la leucine, l'isoleucine, la terleucine, la norleucine, la néopentylglycine, la sérine, la cystéine, la méthionine, l'hexahydrophénylalanine, l'hexahydrophénylglycine, la phénylglycine, la phénylalanine, la tyrosine, l'homophénylalanine, le tryptophane, la naphtylalanine ou la naphtylglycine.

**4.** Dipeptides optiquement actifs polymérisables selon la revendication 1, dans lesquels il s'agit des dipeptides choisis parmi le groupe comprenant l'ester N-méthacryloyl-S-Leu-S-Ile-O-t-butylique, l'ester N-méthacryloyl-S-Leu-S-Phe-d-menthylique, le N-méthacryloyl-S-Leu-S-Leu-3-pentylamide, le N-méthacryloyl-S-Val-S-Val-t-butylamide, l'ester N-acryloyl-S-Phe-S-Phe-2-propylique, l'ester N-méthacryloyl-S-Val-S-Ala-l-bornylique, le N-méthacryloyl-S-Ala-S-Met-3-pentylamide, l'ester N-méthacryloyl-R-Phg-S-Val-t-butylique, l'ester N-méthacryloyl-S-Leu-R-Phe-l-menthylique, le N-méthacryloyl-S-Ala-R-(S)-méthyl-Cys-diéthylamide, l'ester N-méthacryloyl-S-Ala-S-Leu-d-menthylique.

**5.** Procédé pour la préparation des dipeptides optiquement actifs polymérisables selon la revendication 1, caractérisé en ce qu'on lie

A) des dérivés dipeptidiques de formule (II)

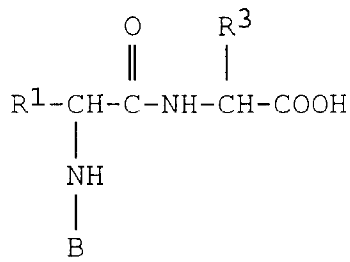

(II)

dans laquelle

$R^1$ et $R^3$ ont la signification indiquée ci-dessus et

B représente un groupe qui se sépare aisément, habituel dans la chimie des peptides,

avec le radical $XR^4$ dans des réactions de liaison habituelles dans la chimie des peptides,
où X et $R^4$ ont la signification indiquée ci-dessus,
et on élimine ensuite le radical B avec des procédés habituels dans la chimie des peptides, puis on fait réagir les composés obtenus répondant à la formule générale (III)

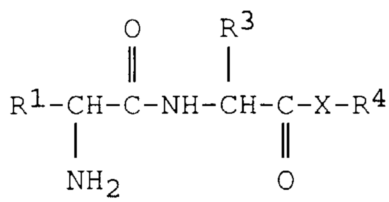

(III)

éventuellement sous forme de leurs produits d'addition d'acides,
avec des dérivés d'acryloyle répondant à la formule (IV)

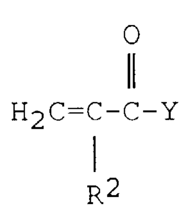

(IV)

dans laquelle

$R^2$ a la signification indiquée ci-dessus, et

Y représente un atome de fluor, un atome de chlore, un atome de brome ou encore le radical $-OCO-CR^2=CH_2$,

en présence d'un agent neutralisant les acides, dans des solvants organiques inertes pour obtenir des composés de formule (I),
ou bien

B) on lie des dérivés d'aminoacides de formule (V)

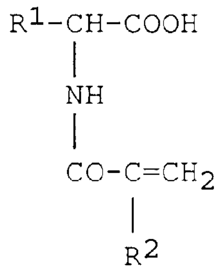

(V)

dans laquelle

$R^1$ et $R^2$ ont la signification indiquée ci-dessus,

avec un second dérivé d'aminoacide de formule (VI)

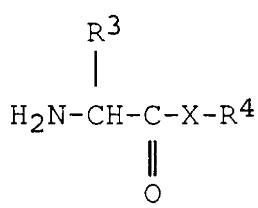

(VI)

dans laquelle

$R^3$, Y et $R^4$ ont la signification indiquée ci-dessus,

conformément à des procédés habituels pour obtenir des dipeptides de formule (I).

**6.** Polymères et copolymères optiquement actifs que l'on obtient par polymérisation, respectivement par copolymérisation des dipeptides optiquement actifs selon la revendication 1.

**7.** Polymères et copolymères optiquement actifs selon la revendication 6, caractérisés en ce qu'ils contiennent des unités de structure de formule (VII)

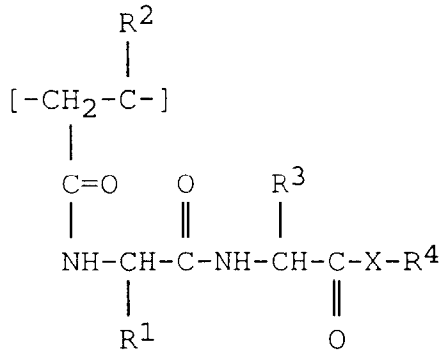 (VII)

à concurrence d'au moins 40 moles %.

**8.** Utilisation des dipeptides optiquement actifs selon la revendication 1, pour la préparation de polymères et de copolymères optiquement actifs.

**9.** Utilisation des polymères et des copolymères optiquement actifs selon la revendication 6, pour le dédoublement chromatographique de mélanges racémiques en antipodes optiques.

**10.** Utilisation des polymères et des copolymères optiquement actifs selon la revendication 6, pour le dédoublement chromatographique de dérivés d'hexahydrocarbazole tels que par exemple le 3-r-(4-fluorophénylsulfonamido)-9-(2-carboxyéthyl)-1,2,3, 4,4a, 9a-hexahydrocarbazole (rac. I), des benzodiazépines telles que par exemple l'Oxazépam (rac. II), des acides arylpropioniques tels que par exemple l'acide 2-(4-isobutylphényl)-propanoïque (rac. III), l'acide 2-(3-benzoyl)-propanoïque (rac. IV), des dérivés de la dihydropyridine tels que par exemple l'ester 5-méthylique de l'acide 1,4-dihydro-2,6-diméthyl-4-(2-trifluorométhylphényl)-pyridine-3,5-dicarboxylique (rac. V), l'ester 3-isopropyl-5-(2-méthoxyéthylique) de l'acide 1,4-dihydro-2,6-diméthyl-4-(3-nitrophényl)-3,5-dicarboxylique (rac. VII, le Chlorthalidon (rac. VIII, des biaryles tels que par exemple le 2,2'-diméthoxy-6,6'-dinitro-biphényle (rac. VIII), des acides cyclopropanecarboxyliques tels que par exemple l'acide cis-3-[2-chloro-4-(2-chlorophényl) éthényl]-2,2-diméthylcyclopropane-carboxylique (rac. IX).